# EUROPEAN PATENT APPLICATION

(11) **EP 4 035 634 A1**
(43) Date of publication of application: **03.08.2022**
(21) Application number: 22153524.8
(22) Date of filing: 26.01.2022
(51) Int. Cl.: A61F 9/008

(54) **LASER CORNEAL PHOTOABLATION METHOD AND SYSTEM FOR TREATING DRY EYE DISEASE**

(30) Priority: 27.01.2021 KR 20210011416
(71) Applicant: WellC, Busan (KR)
(72) Inventor: PARK, Ki Sung, Gyeonggi-do (KR)
(74) Representative: Grosse, Felix Christopher

(57) **Abstract**

A laser corneal photoablation system in accordance with the present invention comprises a corneal measurement unit configured to measure a cornea and obtain state data of the cornea, a laser control unit configured to control an output intensity and range, an amount, and time of a laser according to a photoablation position and a photoablation shape of the cornea based on a corneal photoablation plan generated based on the state data of the cornea, and a laser module 40 configured to irradiate the cornea with a laser according to a control signal applied by the laser control unit, wherein the state data of the cornea includes a corneal surface elevation map, and the laser corneal photoablation system treats dry eye disease by performing corneal photoablation on a surface elevation deviation portion of the cornea.

## Description

The present invention relates to a laser corneal photoablation method and system and more particularly, to a method and system for photoablating a surface elevation deviation portion, which is a surface protrusion of a cornea, to treat dry eye disease.

This patent application was supported by a grant from the Korea Institute for Advancement of Technology (KIAT) of the Ministry of Trade, Industry and Energy (MOTIE). The project number is B0080908000351, the research project is Support for Globalization of Ophthalmic Optical Medical Devices, and the research project name is an individual national application for LASER CORNEAL PHOTOABLATION METHOD AND SYSTEM FOR TREATING DRY EYE DISEASE. The contribution ratio is 1/1, and the name of the institution that carries out the project is Chosun University Industry-University Cooperation Foundation. The research period is 2021.12.01-2022.06.30.

Dry eye disease (DED) or dry eye syndrome is a very common symptom that occurs in more than 300 million people worldwide and is a diagnosis commonly seen in ophthalmology, but it is difficult to define and there is no unique diagnostic test. Recently, the International Dry Eye Work Shop (DEWS) defined dry eye disease as a multifactorial disease of the tears and ocular surface that results in symptoms of discomfort, visual disturbance, and tear film instability along with damage to the ocularsurface.

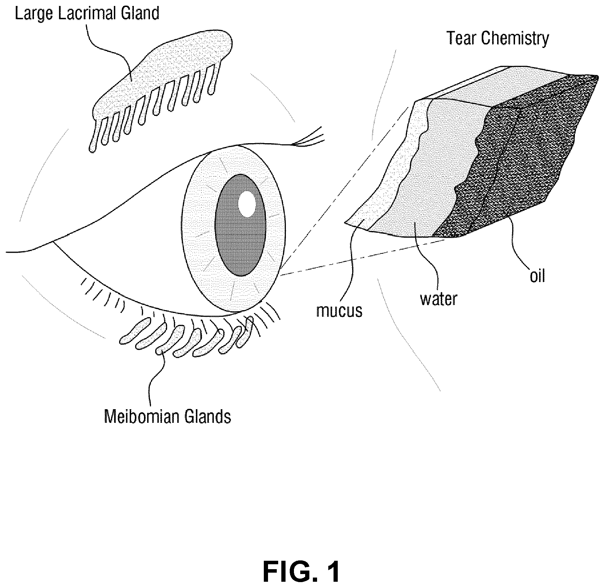

Referring to Fig. 1, tears are made up of a mucus layer, a water layer, and an oil layer constituting the tear film starting from the surface thereof.

In conventional ophthalmology, tear deficiency was considered the main cause of dry eye disease, and tear deficiency was classified by whether mucus produced by conjunctival cells was insufficient, the release of the amount of tears was insufficient, the amount of oil produced by meibomian glands was insufficient, or the oil produced by meibomian glands were not delivered to the tear layer due to blocked meibomian glands, according to the constituents of tears, and treatment methods for dry eye disease were created in the direction toward supplementing and improving the issues described above.

As a practical example, if mucus deficiency is the cause, keratitis, conjunctivitis, and inflammation of the eyelids are treated to increase the amount of mucus secretion in the conjunctiva, and if the deficiency of water content is the cause, artificial tears are added to replenish the tears. If oil deficiency is the cause, the symptoms are relieved by using thermotherapy with an infrared lamp, a warm wet towel, or an IPL laser to facilitate oil release from the meibomian glands.

With respect to the IPL treatment method, U.S. Patent Publication No. 2020-0179168 relates to an eye treatment device comprising a scleral shield positionable proximate an inner surface of an eyelid, and discloses a configuration for applying light energy to a target tissue region to improve meibomian gland dysfunction (MGD). In other words, dry eye disease is treated by irradiating the IPL laser to the surface of the eyelid where the meibomian glands are located to melt meibum with heat, thereby enabling smooth flow of oil.

However, for patients with dry eye disease, there are cases in which symptoms continue despite these conventional methods, such as drug treatment, tear replenishment, and thermotherapy. In this case, it was considered that the cause of dry eye disease was not due to an insufficient supply of tears or oil, but due to over-evaporation of tears coated on the protrusion of the corneal surface.

In fact, ophthalmic surgery, such as cataract surgery or refractive error correction surgery such as myopia or astigmatism, in which the cornea is photoablated with a laser, does not adversely affect the mechanism of tear production and release. Nonetheless, it is quite common for healthy people who did not have dry eye disease before surgery to show symptoms of both vision acuity abnormalities and dry eye disease as postoperative sequelae. According to a featured article in the New York Times, which analyzed the onset of dry eye disease and vision acuity abnormalities after laser refractive correction surgery based on the data published in SCI papers, it was confirmed that in the United States, about 20-40 % of patients experienced sequelae of dry eye disease and visual acuity abnormalities after laser refractive surgery.

Therefore, if these patients do not get better even after using existing treatments for more than 6 months, a more fundamental treatment should be sought. However, until now, only existing treatments that improve the supply of tears and oil are used even for the treatment of dry eye disease resulting from the sequelae of ophthalmic surgery, but until recently, a quantitative treatment method for removing the protrusion on the corneal surface, which resolves the reduction of tears and the oil component constituting the tear film caused by the protrusion on the corneal surface, has never been presented.

The present inventor intends to photoablate the cornea with an excimer or femtosecond laser being used in existing ophthalmology as a means of removing the protrusion on the corneal surface. Current ophthalmic excimer or femtosecond lasers are used only for the purpose of correcting refractive errors by photoablating the cornea or for the incision of the lens capsule or lens during cataract surgery. In the case of IPL lasers that have recently begun to be used in ophthalmology, treatment is carried out that irradiates the eyelids at a low level to generate heat, melts the oil filled in the meibomian gland, and makes it easier to be released to the eyelids, to improve the function of the meibomian gland as in the description of the US patent set forth in the above.

Considering the above situation, it can be inferred that the ophthalmological academia is still not yet aware of the possibility of causing dry eye disease by abnormalities in the shape of the corneal surface, or even if it knows, it is not aware of a method capable of treating dry eye disease by improving the abnormalities in the shape of the corneal surface. Therefore, no one has yet tried the treatment of dry eye disease in terms of correcting the shape of the cornea.

The present invention is designed to improve the shape of the cornea by quantitatively removing the protrusion of the cornea, and it is an object of the present invention to provide a laser corneal photoablation method and system for fundamentally resolving dry eye disease caused by an abnormality in the shape of the corneal.

It is another object of the present invention to provide a laser corneal photoablation method and system for performing refractive correction in conjunction to offset the refractive power of the eye changed due to photoablating the protrusion of the corneal for treating dry eye disease.

In accordance with the present invention, a laser corneal photoablation system comprises a corneal measurement unit configured to measure a cornea and obtain state data of the cornea, a laser control unit configured to control a laser module according to a corneal photoablation plan generated based on the state data of the cornea, and the laser module configured to irradiate the cornea with a laser according to a control signal applied from the laser control unit. The corneal photoablation plan includes a photoablation position and a photoablation shape of the cornea, and the laser control unit controls an output intensity, an output amount, time, and an irradiation position of the laser appropriate for the photoablation position and the photoablation shape of the cornea.

The state data of the cornea includes a corneal surface elevation map, and dry eye disease is treated by performing corneal photoablation on a surface elevation deviation portion of the cornea defined as a protrusion in the present invention.

The corneal surface elevation map preferably comprises a BFS, which is a best-fit sphere, of a corneal surface and elevation information of the corneal surface based on the BFS.

The surface elevation deviation portion of the cornea is an area in which a corneal surface elevation has a predetermined or higher elevation based on the BFS (best-fit sphere), and the corneal photoablation plan comprises information for photoablating the surface elevation deviation portion with a laser for the purpose of causing the surface elevation deviation of the cornea to approximate the BFS.

In addition, the corneal photoablation plan is preferably generated by a calculation means mounted on a corneal photoablation system or a remote calculation means.

In accordance with another aspect of the present invention, there is provided a laser corneal photoablation method performed by a laser corneal photoablation system.

This method comprises providing the laser corneal photoablation system; collecting state data of a cornea, including a corneal surface elevation map; generating, by a calculation means, a corneal photoablation plan for treatment of dry eye disease based on the corneal surface elevation map; and photoablating a surface elevation deviation portion of the cornea so that a surface elevation of the cornea approximates a BFS, by irradiating the cornea with a laser beam according to the corneal photoablation plan for treatment of dry eye disease.

By photoablating the surface elevation deviation portion, dry eye disease caused by a local shape abnormality is treated.

The laser corneal photoablation method preferably further comprises: calculating a corneal photoablation plan for correcting a corneal curvature to the BFS by photoablating the corneal surface elevation deviation; and performing laser refractive correction to prevent the curvature of the cornea from changing to the BFS due to the photoablation of the surface elevation deviation portion. The laser refractive correction proceeds in a manner of eliminating the curvature equal to a difference value obtained by subtracting the curvature value of the original cornea from the curvature value of the BFS.

In accordance with one embodiment of the present invention, the generating a corneal photoablation plan for treatment of dry eye disease comprises processes of generating a photoablation plan for matching the corneal curvature to the BFS by photoablating the corneal surface elevation deviation portion, and performing laser photoablation on the cornea. And, it may further comprise generating an integrated corneal photoablation plan that adds a process of generating a laser photoablation plan for refractive correction that returns the corneal curvature that has changed to the BFS to the corneal curvature before the photoablation to the photoablation plan, and the laser photoablation may be performed on the cornea.

The method also further comprises identifying the surface elevation deviation portion of the cornea in the corneal surface elevation map based on the BFS, wherein the surface elevation deviation portion of the cornea is preferably an area in which a corneal surface elevation has a predetermined or higher elevation based on the BFS (best-fit sphere).

In accordance with another aspect of the present invention, there is provided a method of generating a laser corneal photoablation plan for treatment of dry eye disease, performed by an information processing device.

The method of generating a laser corneal photoablation plan for treatment of dry eye disease comprises collecting state data including a corneal surface elevation map requiring treatment of dry eye disease; determining a treatment area for dry eye disease based on a surface elevation deviation based on a BFS of a cornea in the collected corneal surface elevation map; and generating a corneal photoablation plan for photoablating so that an elevation of the treatment area for dry eye disease of the cornea approximates an elevation of the BSF.

The method of generating a laser corneal photoablation plan preferably further comprises: determining the treatment area for dry eye disease as a photoablation target area if a surface elevation of the cornea is 20 µm or higher based on the BFS in the corneal surface elevation map, in that dry eye disease treatment begins when the thickness of the tear layer coated on the corneal surface becomes thinner than 20 µm in ophthalmology.

In accordance with another aspect of the present invention, the method of generating a laser corneal photoablation plan comprising the above steps may be implemented as a computer program consisting of instructions for executing this method, and this program may be stored on a computer-readable storage medium.

According to the technical configuration described above, there is provided a laser corneal photoablation method and system that fundamentally resolves dry eye disease caused by an abnormality in the shape of the corneal.

According to another aspect of the present invention, there is provided a laser corneal photoablation method and system that performs correction of dry eye disease and refractive errors.

A more complete appreciation of the invention and many of the attendant advantages thereof will be readily obtained as the same becomes better understood by reference to the following detailed description when considered in connection with the accompanying drawings, wherein:
- Fig. 1: is an illustration showing the structure of the eye and tears;
- Fig. 2: is a block configuration diagram showing the structure of a laser corneal correction system;
- Fig. 3: is a cross-sectional view schematically showing a tear layer, a reference plane, a surface elevation deviation portion, and the surface of the cornea, before and after removing the surface elevation deviation portion;
- Fig. 4: is a diagram schematically showing a BFS and the surface of the cornea;
- Fig. 5: is a diagram schematically showing the distribution of the tear layer and the refractive direction error due to the surface elevation deviation portion of the cornea, and the distribution of the tear layer (dotted line) and a corrected state of the refractive direction after the surface elevation deviation portion has been removed, with the assumption that the thickness of the tear layer (dotted line) distributed on the cornea is 20 microns;
- Fig. 6: is a quad map of the cornea of Embodiment 1 photographed and analyzed by an anterior segment tomography (Bausch and Lomb Orbscan IIz Corneal Analysis System);
- Fig. 7: is a cross-sectional view when the quad map of Fig. 6 is cut along the line of 0-180 degrees from the cross-sectional view of the corneal surface elevation map located in the upper left, the BFS of the cornea and its corresponding corneal surface elevation deviation can be easily ide ntified;
- Fig. 8: is a corneal surface elevation map that appears before and after photoablating the surface elevation deviation portion with a laser;
- Fig. 9: is a corneal quad map of Embodiment 2 photographed and analyzed by an anterior segment tomography (Bausch and Lomb Orbscan IIz Corneal Analysis System);
- Fig. 10: is a cross-sectional view of the corneal surface elevation map (front view) of Fig. 9 cut from 0 degrees to 180 degrees; and
- Fig. 11: is a corneal surface elevation map before and after photoablating the surface elevation deviation portion of Embodiment 2 with a laser.

Hereinafter, a laser photoablation method and a system thereof in accordance with an embodiment of the present invention will be described with reference to the accompanying drawings. In this process, the thickness of the lines, the size of the components, or the like shown in the drawings may be exaggerated for the sake of clarity and convenience of explanation. In addition, the terms to be described below are terms defined in consideration of the functions in the present invention, which may vary according to an operator, the intention of the operator, or practice. Therefore, definitions of these terms should be made based on the content throughout this specification.

In the structure of the tear shown in Fig. 1, the innermost mucus layer in contact with the cornea is the thinnest and is produced by the cells in the conjunctiva (the transparent epidermis surrounding the eye), and the mucus layer helps the overlaying water layer spread evenly over the eye. The water layer (intermediate layer) is essentially a very dilute saline solution, and is the largest and thickest. The water layer helps to expel dust, dirt, or other foreign substances that can get into the eye out of the eye, keeping the eye moist and comfortable at all times. The lacrimal gland and accessory tear gland under the upper eyelid produce this water layer, and any issue with this water layer is the most common cause of dry eye disease, and a disease resulting therefrom is called keratoconjunctivitis sicca (KCS).

The outermost layer of the tear is a very thin oil layer, and the oil is produced by the meibomian gland and the Glands of Zeis. The main function of the oil layer is to be located on the surface of the tear and to protect the underlying water layer beneath it, and to prevent dryness of the cornea by increasing the evaporation time of the tears coated on the cornea.

With respect to an ophthalmic laserto be used as an implementation tool of the present invention, laser-applied surgical methods performed on the human eyes include several different types of surgery, and are aimed at improving eyesight, treating eye diseases, or both. Commonly known types of surgery include LASIK (laser in-situ keratomileusis) or LASEK (laser epithelial keratomileusis), corneal transplantation (keratoplasty), intracorneal lenticule extraction, intracorneal ring segment insertion, corneal inlay transplantation, cataract surgery, etc.

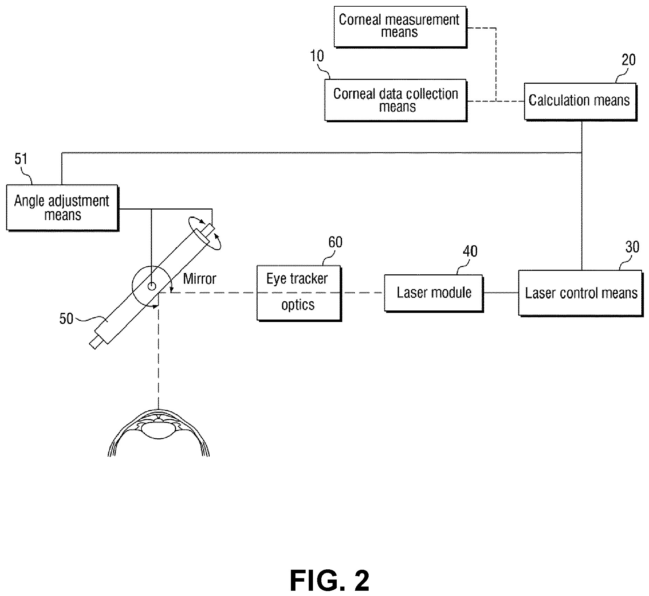

Referring to Fig. 2, the laser corneal photoablation system includes a corneal measurement unit, a calculation means 20 configured to establish a photoablation plan based on corneal state information, a laser control unit 30, a laser module 40, and an optical system 50. The laser corneal photoablation system may further include a corneal information collection unit 10 configured to collect patient corneal state information and/or other patient information measured by the corneal measurement unit.

The corneal measurement unit is an optical device that can measure the state of the cornea, such as a Cornea Analysis Device or an eye imaging device, the calculation means is an information processing device such as a computer, and the laser control unit includes a circuit or information processing device for controlling the laser module to irradiate the eyeball with the laser according to the photoablation plan calculated by the calculation means.

The laser module is a laser generator including a laser source such as an excimer laser, a femtosecond laser, etc., and various ancillary equipment, and the optical system 50 includes a lens, a mirror, an optical fiber, etc., that transmit laser light from the laser module to the laser irradiation portion of the cornea. The system may further include a monitoring unit for monitoring the laser light and the eyeball in real-time, an eye tracker for tracking the eyeball, and an optical system drive unit.

These laser corneal photoablation systems have been used for refractive correction, such as LASIK or LASEK.

On the other hand, in the structure of the tear in Fig. 1, the thickness of the tear layer coated on the corneal surface differs from scholar to scholar claiming that its average is 25 microns or it is of 20-40 microns, but what they have in common is that the thickness of the tear layer covering the protrusion on the corneal surface is inevitably thinner than the flat part around the protrusion. Regarding the diagnosis of dry eye disease, in the case of OCULUS Keartograph 5M (Germany), which is a measuring device for dry eye disease, the tear thickness is measured and if the thickness of the tear layer is 20 microns or less, it is determined that there are dry eye symptoms, and treatment is started based on this in ophthalmology.

The present inventor has focused on structural issues that due to the nature of tears, the protruding portion with a higher corneal surface elevation hinders even coating of tears on the corneal surface, has thinner tears coated on its surface compared with that of other flat parts, as well as has tears flowing down faster than in portions with a lower corneal surface elevation or insignificant protrusions, causing the corneal surface to dry faster. That is, despite the irrelevance with lack of tears or meibomian gland abnormalities, drying of the cornea due to local over-evaporation of water coated on the cornea and the fact that dry eye disease is caused therefrom, in addition, the fact that young people who were irrelevant with dry eye disease continue to suffer from visual acuity abnormalities and dry eye disease after the cornea has been photoablated in laser refractive correction, and dry eye disease along with visual acuity abnormalities after trabeculectomy surgery for cataract or glaucoma, which does not have causal factors for dry eye disease have led to continued treatments. In addition, the fact that as the number of patients suffering from long-term dry eye disease treatment continues to increase, the market size of dry eye disease treatment out of the ophthalmic treatment market in Korea is growing by more than 6 % per year and the market size continues to expand, and the like have led to the thought that it was necessary to develop another method as a treatment technique for dry eye disease other than the existing method of replenishing only tears, and then to perform treats independently or in combination with the existing methods.

Therefore, the mechanism of dry eye disease being caused or aggravated by corneal surface elevation deviation will be described, and it is intended to photoablate, lower, or remove the corneal surface elevation deviation portion with a laser to prevent the cornea from being dry and improve dry eye symptoms.

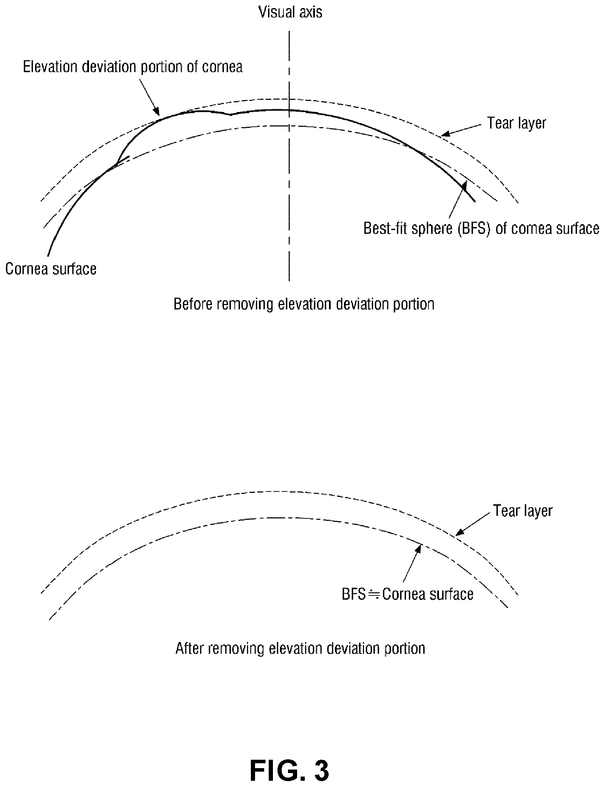

Fig. 3 is an exemplary illustration schematically showing a cross section of the corneal surface and the tear layer (dotted line) exaggeratedly for the convenience of description. In Fig. 3, a reference plane (dash-dotted line) in elevation of the corneal surface is a best-fit sphere (BFS), and refers to an optimal spherical surface that best fits the surface elevation of the cornea. Part of the corneal surface may protrude locally with an elevation different from the reference plane, and this elevation deviation portion is indicated by a solid line. In the case of other corneas, if the surface protrusion is insignificant or generally coincides with or is approximate to the reference plane, tears are evenly distributed over the entire corneal surface as shown by the dotted line in Fig. 3. In this case, the possibility that dry symptoms arise in the cornea is slim.

Even if the volume of tears with a thickness of about 20-50 microns consisting of the mucus layer, the water layer, and the oil layer as shown in Fig. 3 is not insufficient, it is difficult to keep the tears uniform in the elevation deviation portion that protrudes convexly from the reference plane (flat portion). This is because, in the surface elevation deviation portion (protrusion), the coated tears flow down at a faster rate than in the reference plane (flat portion) and its surface dries quickly.

Therefore, even if there is no problem in the supply of tears to be coated on the cornea, tears cannot be maintained for an appropriate time in and around the portion where the cornea protrudes, and thus, dry eye disease occurs.

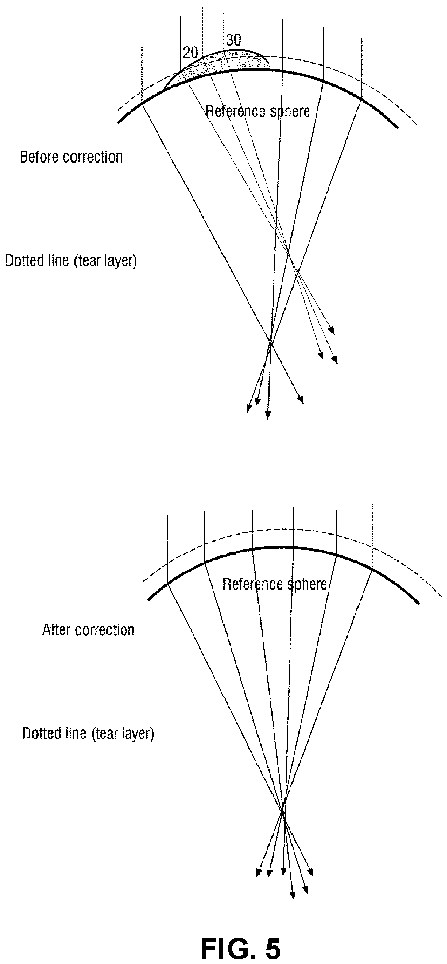

In addition, the portion where the cornea protrudes causes an error in the direction of refraction different from that of the surrounding flat portion, thereby partially reducing the sharpness of the visual acuity. Accordingly, if the surface elevation deviation portion of the cornea is photoablated, the corneal curvature becomes similar or equal to that of the flat portion and the refraction deviation occurred in the protrusion decreases or disappears, thereby contributing to the improvement of clear visual acuity as well. A sketch related to the error in the direction of refraction due to such a refraction deviation is schematically illustrated in Fig. 5.

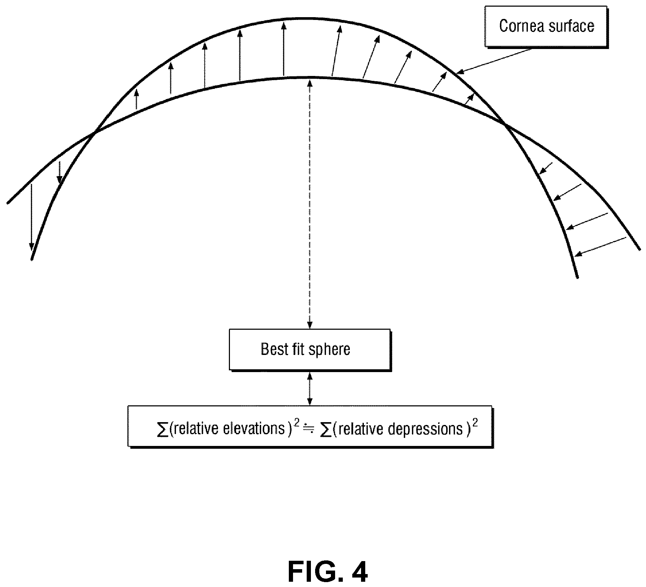

On the other hand, Fig. 4 is a cross-sectional view illustrating the concept of BFS (best fit sphere) for the curved surface formed by the corneal surface, and the BFS is defined to refer to an imaginary sphere that matches as closely as possible to the surface height (surface elevation) of the entire cornea measured. Thus, the surface of the cornea beyond the imaginary sphere, the BFS, is minimized. In Fig. 4, the yellow line is the BFS, and the green line is the surface of the cornea. The red arrow indicates a deviation by which the cornea is higher than the BSF in elevation, and the blue arrow indicates a deviation lower than the BSF in elevation. The sum of the squares of the deviations in higher elevation is approximately equal to the sum of the squares of the deviations in lower elevation. Therefore, the sum of the higher and lower deviations of the corneal surface deviating from the imaginary sphere BFS is minimized.

The mathematical calculation for the BSF is determined by a mathematical method such as the one described in Corneal Elevation Topography: Best Fit Sphere, Elevation Distance, Asphericity, Toricity, and Clinical Implications, Cornea: May 2011 - Volume 30 - Issue 5 -p 508-515 by doi: 10.1097/ICO.0b013e3181fb4fa7, with the authors of Gatinel, Damien MD, PhD; Malet, Jacques PhD; Hoang-Xuan, Thanh MD; Azar, Dimitri T MD, et al.

Figs. 3 and 5 can describe the phenomenon well, in which visual acuity abnormality and dry eye disease appear simultaneously due to the distribution of the tear layer and the refractive error in the protruding portion.

In orderto resolve such a problem, the inventor of the present invention presents a method for improving dry eye disease by lowering or removing the surface elevation deviation portion of the cornea.

That is, by reducing the corneal surface elevation deviation portion and making it the same as or close to the BSF as shown in Fig. 3, the height of the tear layer (dotted line) coated on the cornea is not insufficient locally in particular portions, and spreads evenly over the cornea to block corneal dryness. This helps alleviate or eliminate the symptoms of dry eye disease. In addition, the refractive error that occurred in the corneal surface elevation deviation portion can be reduced or eliminated as well, and thus, the visual acuity abnormality can also be improved.

The laser corneal photoablation method proposed in the present invention to achieve the above goal is configured such that first, by photoablating the corneal surface elevation deviation portion with a laser and thereby making the tear distribution even on the cornea, dry eye disease caused by corneal dryness due to the surface elevation deviation portion of the corneal surface is treated, and second, the laser refraction correction is performed in conjunction so as to correct (offset) the refractive power of the eye that changes from before the photoablation due to the corneal curvature that becomes close to or coincides with the BFS as the laser photoablates the corneal surface elevation deviation portion. The advantage of this method is that if the method is performed, it can be performed in combination with the conventional laser refractive correction surgery if the operatee wants.

In the following, a laser corneal photoablation method performed by the laser corneal photoablation system of the present invention will be described in detail.

First, a laser corneal photoablation system is provided.

A corneal measurement unit device such as a cornea analysis device or a cornea imaging device measures the state of the cornea.

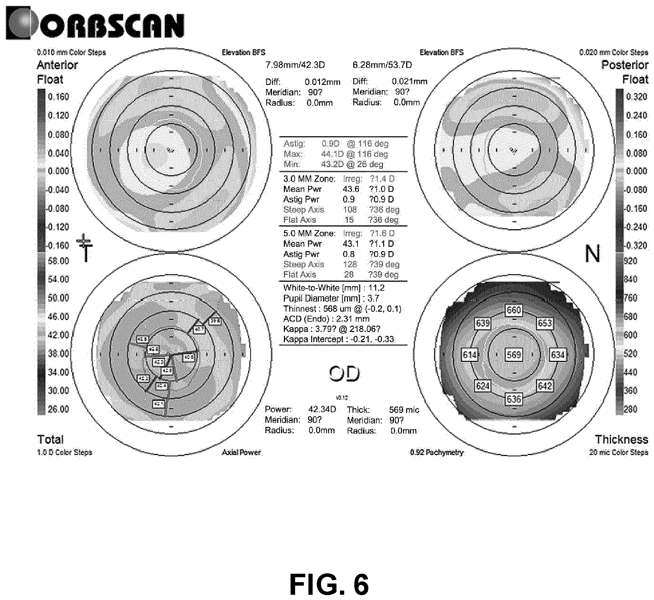

Fig. 6 is an elevation map of the cornea photographed and analyzed by an anterior segment tomography (Bausch and Lomb Orbscan IIz Corneal Analysis System). The upper left of Fig. 6 is a corneal surface elevation map, the upper right is a posterior elevation map of the cornea, the lower left is a curvature map of the cornea, and the lower right is a thickness map of the cornea.

In the corneal surface elevation map located in the upper left of Fig. 6, the color bar of the corneal surface elevation map showing the surface elevation deviation of the cornea based on the best-fit sphere shows the elevation, the green color is the area (elevation of 0 microns) having the same height as the BFS defined by the anterior segment tomography serving as the reference, the red and yellow parts are areas higher than the BFS, and the blue and purple parts are areas lower than the BFS.

After that, the corneal information collection unit collects and transmits corneal state information including the corneal surface elevation map measured by the corneal measurement unit in real-time and other patient state information to the calculation means. The calculation means generates a corneal photoablation plan for the treatment of dry eye disease based on the corneal surface elevation map.

The corneal surface elevation map is a map showing the BFS and a corneal surface elevation deviation using the BFS as a reference plane. The area where the thickness of the tear layer has an elevation of about 20 µm or more relative to the BFS is an area where tears can dry out and cause dry eye disease due to corneal dryness, and this part is determined as a target area for corneal photoablation for treating dry eye disease. If there is no target area for photoablation, it is determined that the cornea is not dry eye disease due to a local shape abnormality. However, if an area where the thickness of the tear layer has an elevation of about 20 µm or less relative to the BFS is also determined to be a potential area where tears can dry out and cause dry eye disease due to corneal dryness, this part may also be determined as a target area for corneal photoablation for treating dry eye disease.

The "surface elevation deviation portion of the cornea that is photoablatable" with a refractive correction laser is the part where the actual corneal surface is higher than the BFS curvature defined by the ophthalmic measurement device such as a cornea analysis device or anterior segment imaging device, the photoablatable area of the cornea is an 8.5 mm zone centered on the visual axis of the cornea, and the size of this area is the same as the general photoablation area being used for cornea photoablation by the ophthalmic refractive correction laser during the refractive correction. However, it may be increased to a wider area of up to 10.5 mm zone if necessary.

If the calculation means extracts the surface elevation deviation portion of the cornea and determines it as a dry eye disease treatment area, it generates a corneal photoablation plan for the treatment of dry eye disease, and does not generate a photoablation plan for the treatment of dry eye disease in areas other than the dry eye disease treatment area.

The corneal photoablation plan for the treatment of dry eye disease is to photoablate and remove the surface elevation deviation portion of the cornea with a laser so as to be equal to or close to the best-fit sphere (BFS). To this end, information such as specific photoablation target coordinates, the amount of tissue photoablation at the corresponding coordinates, the type of photoablation and laser intensity therefor, laser irradiation time, etc., may be generated together as part of the corneal photoablation plan for the treatment of dry eye disease.

According to the corneal photoablation plan for the treatment of dry eye disease generated by the calculation means, the laser control unit performs corneal photoablation by controlling the laser output intensity and shape, irradiation position, time, and irradiation amount.

The laser corneal photoablation system includes an eye tracker that tracks the position of the eyeball and cornea in real-time for accurate photoablation, and a laser light delivery unit including various drive devices and optical systems.

After the local surface elevation deviation portion of the cornea has been removed and the anterior surface of the cornea has been corrected to the BFS as shown in Fig. 4 by the laser corneal photoablation according to the photoablation plan for the treatment of dry eye disease, the refractive power of the eye changes by the difference in the corneal curvature that has changed. Therefore, in order to eliminate the visual discomfort caused by this, it is necessary to offset the change in the refractive power of the eye by correcting with the existing laser refractive correction by the corneal curvature value changed. Regarding the above method, the laser refractive correction step comprises: calculating a corneal photoablation plan for refractive correction to offset the changed refractive power of the eye whose corneal surface elevation has been corrected to the BFS according to the corneal photoablation plan for the treatment of dry eye disease; and performing laser refractive correction according to the corneal photoablation plan.

In a more detailed description of the calculating the corneal photoablation plan for refractive correction, the calculation means is an information processing device including a CPU, etc., and if it obtains the numerical values of "an average curvature value of the cornea measured" by the cornea analysis device or anterior segment tomography before the correction and "a calculated BFS curvature value of the cornea" and then determines the difference value between the two curvature values, it is possible to calculate the difference value in refractive power of the eye that is changed due to corneal photoablation for the treatment of dry eye disease. At this time, the "curvature of the cornea measured" by the anterior segment tomography or the cornea analysis device to be used may be selected autonomously by an ophthalmologist according to the desired treatment goal out of a central 3 mm, 5 mm, or 7 mm zone or an average curvature value of the desired area.

After establishing a refractive correction plan to offset (correct) the refractive power of the eye that has become different between before and after the photoablation by the above method and inputting it to the refractive correction laser, if ① corneal photoablation for the treatment of dry eye disease is performed, followed by ② corneal photoablation for refraction correction in conjunction, then as a result, corneal surface elevation deviation correction without a change in the refractive power of the eye is achieved.

Thereafter, the control means links the laser with the cornea analysis device or anterior segment imaging device, so that the laser automatically removes the surface elevation deviation portion of the cornea, and in conjunction, removes the cornea by the refractive correction value established by the above method. However, in this case, the operator should perform laser photoablation after checking all these processes.

With the above process, it is possible to maintain the refractive power of the eye as it is while removing the elevation deviation portion of the corneal surface that causes dry eye disease with a laser.

In the following, a method of removing the surface elevation deviation portion of the cornea that causes dry eye disease will be described with Embodiments 1 and 2 (simulation).

Both patients developed dry eye disease and tried a treatment of delaying the water evaporation in the cornea for more than six months by performing thermotherapy to improve the function of meibomian glands with an IPL laser along with treatments for tear production and replenishment, but the symptoms have not gotten any better. Therefore, it could be confirmed that the dry eye disease was not due to a lack of tears or water evaporation.

As a result of checking the surface elevation of the cornea with an anterior segment tomography, it was confirmed that there was a protrusion with a surface elevation deviation of 20 microns or more, and it was determined that the dry eye disease resulted therefrom, and laser corneal photoablation for the treatment of dry eye disease was performed as follows.

Fig. 6 is a quad map of the cornea photographed by an anterior segment tomography. The surface elevation deviation of the cornea can be checked with the corneal surface elevation map (the map marked as Anterior Float) located on the upper left side of Fig. 6.

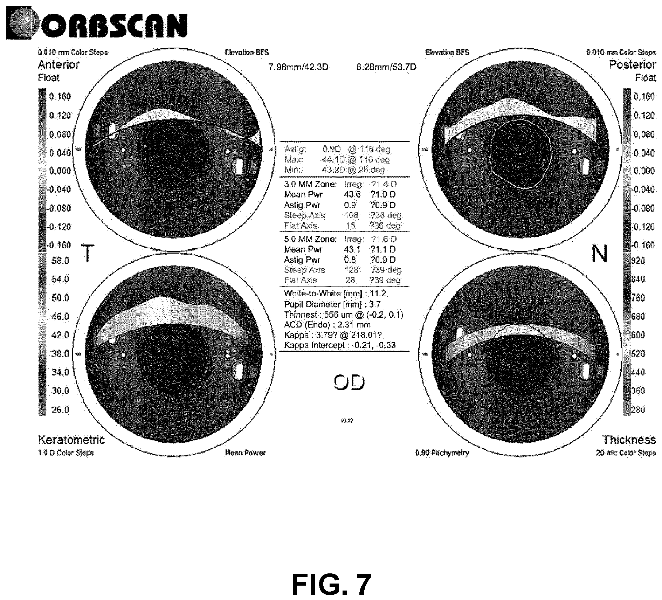

Fig. 7 is a cross-sectional view when the quad map of Fig. 6 is cut along the line of 0-180 degrees. From the cross-sectional view of the corneal surface elevation map located in the upper left of Fig. 7, the BFS of the cornea and the corneal surface elevation deviation corresponding thereto can be easily confirmed.

As shown through the corneal surface elevation map in the upper left of Fig. 6, as a result of extracting and checking the surface elevation deviation portion with the corneal surface elevation map obtained by the calculation device from the corneal measurement unit, it is confirmed that there is a portion (dark yellow) at a surface elevation of 30 µm based on the BFS at a place slightly biased in the direction of 225 degrees from the center of the cornea and a circular surface elevation deviation portion (yellow) at a surface elevation of 20 µm surrounding the portion of the surface elevation of 30 µm thereabout.

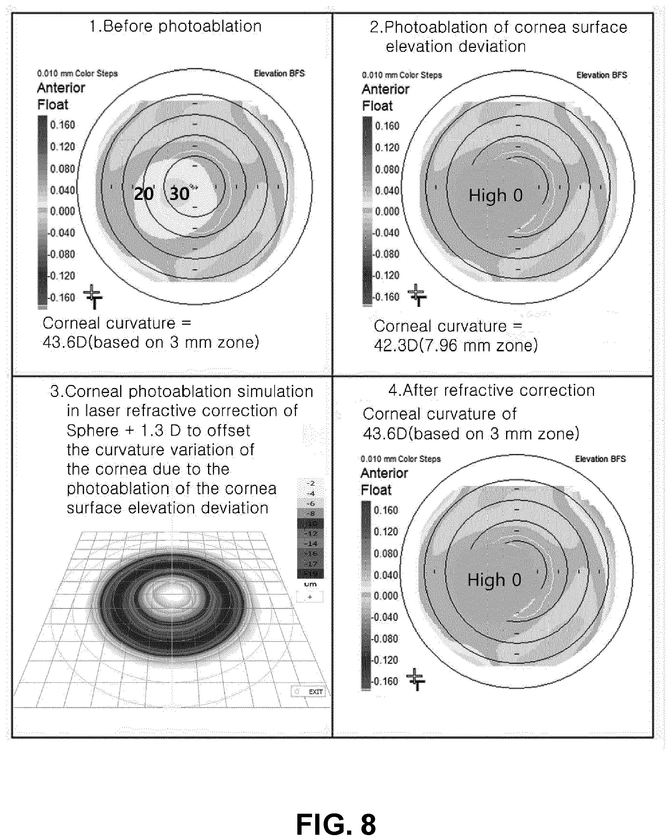

At this time, the calculation device generates a corneal photoablation plan for the treatment of dry eye disease that photoablates the surface elevation deviation portion of the cornea with a laser so as to be equal to the reference plane, BFS, and the laser control unit controls the laser module to photoablate according to the corneal photoablation plan for the treatment of dry eye disease. 1 and 2 of Fig. 8 show a change in the surface elevation deviation relative to the reference plane of the corneal surface elevation map before and after performing the corneal photoablation for the treatment of dry eye disease, and although there are surface elevation deviation portions in the center of the cornea and the area adjacent to it in the left figure, as the surface elevation deviation portions are removed by laser photoablation, it can be seen in the right figure that the corneal surface has changed to a distribution close to the BFS without the surface elevation deviation portions. This enables an even distribution of the tear layer, providing a new horizon for the treatment of dry eye disease.

At this time, in order to offset the refractive power of the eye that is changed by removing the surface elevation deviation of the cornea, laser refractive correction is performed in conjunction. In this case, the calculation of the refractive power to be corrected can be made by a method of subtracting the "corneal curvature determined to be the BFS by the anterior segment tomography" from the "average corneal curvature measured by the anterior segment tomography" before the photoablation. Applying Embodiment 1, the number 43.6 can be confirmed in the Mean Power item written one row below the item written as 3.0 MM Zone under the second black line among the quad map of Fig. 6 or Fig. 7. From this, it can be seen that the average refractive power at the 3 mm zone of the center of the cornea is 42.4 D (diopters). Accordingly, the "average corneal curvature measured before the photoablation" of the corresponding cornea is 43.6 D. The "corneal curvature determined to be the BFS by the anterior segment tomography" can be learned from the information written as 7.96 mm/42.3 D one row below in the right of what is written as Elevation BFS in the upper right of the corneal surface elevation map located in the upper left of Fig. 6 or Fig. 7. The above notation means that the curvature value corresponding to the best-fit sphere (BFS) in the area of the 7.96 mm zone of the center of the cornea is 42.3 D. Therefore, the formula for calculating the refractive power to be corrected becomes 43.6 D (average corneal curvature before photoablation measured by the anterior segment tomography) - 42.3 D (corneal curvature determined to be the BFS by the anterior segment tomography) = +1.3 D (refractive power of the eye changed due to corneal surface elevation correction). Thus, if Sphere +1.3 D is corrected together by the refractive correction laser during corneal surface elevation correction, the corneal surface elevation correction before and after laser photoablation is achieved without a change in the refractive power of the eye. The corneal photoablation pattern of the refractive laser for Sphere +1.3 D correction and the change in the curvature of the cornea after execution can be checked in Figs. 8-3 and 8-4, respectively.

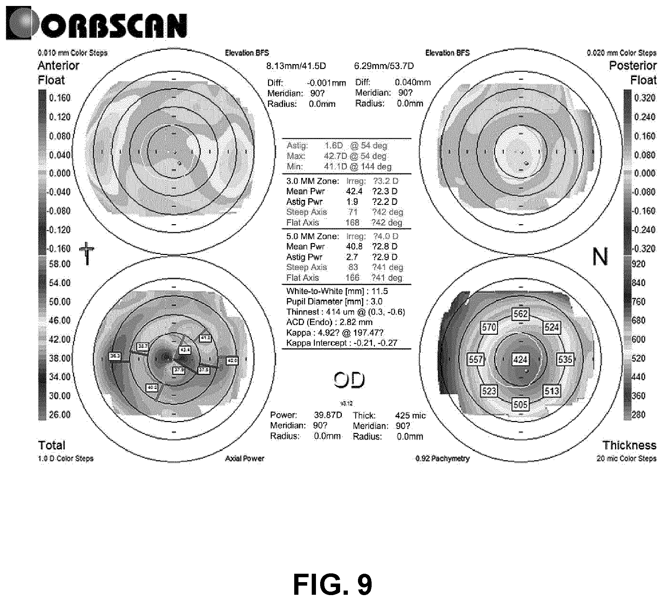

Fig. 9 is a quad map of the cornea photographed and analyzed by an anterior segment tomography. The surface elevation deviation of the cornea can be checked with the corneal surface elevation map (the map marked as Anterior Float) located on the upper left side of Fig. 9.

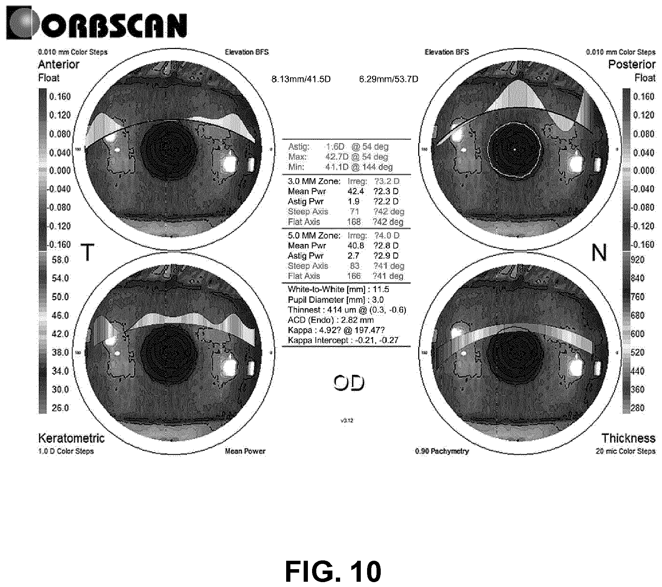

Fig. 10 is a cross-sectional view when the corneal quad map of Fig. 9 is cut along the line of 0-180 degrees. From the cross-sectional view of the corneal surface elevation map located in the upper left of Fig. 10, the BFS of the cornea and the corneal surface elevation deviation corresponding thereto can be easily confirmed.

As shown through the corneal surface elevation map in the upper left of Fig. 9, as a result of extracting and checking the surface elevation deviation portion with the corneal surface elevation map obtained by the calculation device from the corneal measurement unit, it is confirmed that there are portions (dark yellow) at a surface elevation of 30 µm based on the BFS, outside the 6 mm zone on the left and outside the 2 mm zone on the right in the direction of 0 degrees and 180 degrees of the center of the cornea.

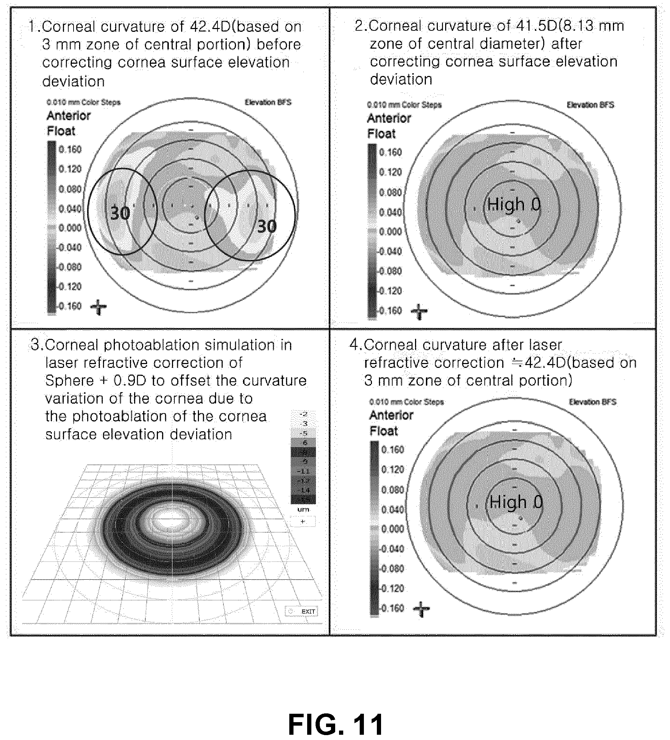

At this time, the calculation device generates a corneal photoablation plan for the treatment of dry eye disease that photoablates the surface elevation deviation portion of the cornea with a laser so as to match the best-fit sphere (BFS), which is the reference plane, and the laser control unit controls the laser module to perform photoablation according to this corneal photoablation plan for the treatment of dry eye disease. Figs. 11-1 and 11-2 show a change in the surface elevation deviation relative to the reference plane of the corneal surface elevation map before and after performing the corneal photoablation for the treatment of dry eye disease, and although there are surface elevation deviation portions in the peripheral areas of the cornea in the direction of 0 and 180 degrees in the left figure, as the surface elevation deviation portions are removed by laser photoablation, it can be seen in the right figure that the corneal surface has changed to a distribution close to the best-fit sphere (BFS) without the surface elevation deviation portions. This allows for an even distribution of the tear layer, providing a new horizon for the treatment of dry eye disease.

When the surface elevation deviation of the cornea is removed as described above, the curvature of the cornea is changed and the refractive power of the eye is also changed. Therefore, it is necessary to perform laser refractive correction, which corrects by the amount of the refractive power changed, together when removing the surface elevation deviation of the cornea, so that there is no change in the refractive power of the eye before and after the corneal photoablation. In this case, the calculation of the refractive power to be corrected can be made by a method of subtracting the "corneal curvature determined to be the BFS by the anterior segment tomography" from the "average corneal curvature measured by the anterior segment tomography" before the photoablation. Applying Embodiment 2, the number 42.4 can be confirmed in the Mean Power item written one row below the item written as 3.0 MM Zone under the second black line among the quad map of Fig. 9 or Fig. 10. From this, it can be seen that the average refractive power at the 3 mm zone of the center of the cornea is 42.4 D (diopters). Accordingly, the "average corneal curvature measured before the photoablation" of the corresponding cornea is 42.4 D. The "corneal curvature determined to be the BFS by the anterior segment tomography" can be learned from the information written as 8.13 mm/41.5 D one row below in the right of what is written as Elevation BFS in the upper right of the corneal surface elevation map located in the upper left of Fig. 9. The above notation means that the curvature value corresponding to the best-fit sphere (BFS) in the area of the 8.13 mm zone of the center of the cornea is 41.5 D. Therefore, 42.4 D (average corneal curvature before photoablation measured by the anterior segment tomography) - 41.5 D (corneal curvature determined to be the BFS by the anterior segment tomography) = +0.9 D (refractive power of the eye changed due to corneal surface elevation correction). Thus, if spherical aberration (Sphere) + 0.9 D is corrected together by the laser during the laser photoablation for corneal surface elevation correction, the corneal surface elevation correction before and after laser photoablation is achieved without a change in the refractive power of the eye. The corneal photoablation pattern of the refractive correction laser for correcting spherical aberration (Sphere) +0.9 D and the change in the curvature of the cornea after refractive correction has been performed can be checked in Figs. 11-3 and 11-4, respectively.

Although the present invention has been described with reference to the embodiments illustrated in the drawings, these are illustrative only, and those of ordinary skill in the art to which the present techniques pertain will appreciate that various modifications and other equivalent embodiments are possible therefrom. Accordingly, the true technical protection scope of the present invention should be defined by the following claims.

### REFERENCE SIGNS

- 10: Corneal data collection means
- 20: Calculation means
- 30: Laser control means
- 40: Laser module
- 50: Mirror
- 51: Angle adjustment means
- 60: Eye tracker optics

## Claims

1. A laser corneal photoablation system comprising a corneal measurement unit configured to measure a cornea and obtain state data of the cornea, a laser control unit configured to control a laser module (40) according to a corneal photoablation plan generated based on the state data of the cornea, and the laser module (40) configured to irradiate the cornea with a laser according to a control signal of the laser control unit,
wherein the state data of the cornea includes a corneal surface elevation map, and the laser corneal photoablation system treats dry eye disease by performing corneal photoablation on a surface elevation deviation portion of the cornea.

2. The laser corneal photoablation system of claim 1, wherein
the corneal surface elevation map comprises a BFS (best-fit sphere) of a corneal surface and elevation information of the corneal surface based on the BFS,
the surface elevation deviation portion is an area in which a corneal surface elevation has a predetermined or higher elevation based on the BFS, and
the corneal photoablation plan comprises information for photoablating the surface elevation deviation portion with a laser so that the corneal surface elevation approximates the BFS.

3. The laser corneal photoablation system of claim 1 or 2, wherein
the corneal photoablation plan is generated by a calculation means (20) mounted on a corneal photoablation system or a remote calculation means (20).

4. A laser corneal photoablation method performed by a laser corneal photoablation system, comprising:
providing the laser corneal photoablation system;
collecting state data of a cornea, including a corneal surface elevation map;
generating, by a calculation means (20), a corneal photoablation plan for treatment of dry eye disease based on the corneal surface elevation map; and
photoablating a surface elevation deviation portion of the cornea so that a surface elevation of the cornea approximates a BFS, by irradiating the cornea with a laser beam according to the corneal photoablation plan for treatment of dry eye disease,
wherein the laser corneal photoablation method treats dry eye disease caused by a local shape abnormality, by photoablating the surface elevation deviation portion with laser light.

5. The laser corneal photoablation method of claim 4, further comprising:
calculating a corneal photoablation plan for refractive correction to offset a changed refractive power of an eye whose corneal surface elevation has been corrected to the BFS according to the corneal photoablation plan for treatment of dry eye disease; and
performing laser refractive correction according to the corneal photoablation plan.

6. The laser corneal photoablation method of claim 4, further comprising:
identifying the surface elevation deviation portion of the cornea in the corneal surface elevation map based on the BFS,
wherein the surface elevation deviation portion is an area in which a corneal surface elevation has a predetermined or higher elevation based on the BFS.

7. A method of generating a laser corneal photoablation plan for treatment of dry eye disease, performed by an information processing device, comprising:
collecting state data including a corneal surface elevation map requiring treatment of dry eye disease;
determining a treatment area for dry eye disease based on a surface elevation deviation based on a BFS of a cornea in the collected corneal surface elevation map; and
generating a corneal photoablation plan for photoablating so that an elevation of the treatment area for dry eye disease of the cornea approximates an elevation of the BSF.

8. The method of generating a laser corneal photoablation plan of claim 7, further comprising:
determining the treatment area for dry eye disease as a photoablation target area if a surface elevation of the cornea is 20 µm or higher based on the BFS in the corneal surface elevation map.
